# FASCICULE DE BREVET EUROPEEN

(11) **EP 2 818 201 B1**
(45) Date de publication et mention de la délivrance du brevet: **20.07.2016**
(21) Numéro de dépôt: 14165463.2
(22) Date de dépôt: 22.04.2014
(51) Int. Cl.: A61N 1/375, A61N 1/372, A61N 1/05

(54) **Capsule intracardiaque et accessoire d'implantation in situ par voie fémorale**
Intrakardiale Kapsel und entsprechendes Zubehörteil zur Implantation in situ über den femoralen Weg
Intracardiac capsule and accessory for in situ implantation by the femoral route

(30) Priorité: 24.06.2013 FR 1356020
(43) Date de publication de la demande: 31.12.2014
(73) Titulaire: Sorin CRM SAS, 92140 Clamart Cedex (FR)
(72) Inventeur: Ollivier, Jean-François, 91190 Villiers Le Bacle (FR)
(74) Mandataire: Dupuis-Latour, Dominique

(56) Documents cités:
- EP-A1- 2 394 695
- US-A1- 2007 135 883
- US-A1- 2009 204 170
- US-A1- 2012 095 539

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation, de cardioversion et/ou de défibrillation en cas de trouble du rythme détecté par le dispositif.

L'invention concerne, de façon générale, l'implantation *in situ* de tels dispositifs pourvus à leur extrémité distale d'un organe d'ancrage du dispositif, apte à pénétrer dans les tissus d'une paroi corporelle au site d'implantation choisi.

Un exemple typique d'un tel organe d'ancrage est une vis hélicoïdale saillante prolongeant axialement le corps du dispositif médical et destinée à pénétrer dans le tissu cardiaque par vissage au site d'implantation. Ce mode d'ancrage n'est toutefois pas limitatif de l'invention, qui s'applique également à d'autres types d'organes d'ancrage, par exemple mettant en oeuvre des aiguilles, des crochets, des barbes, etc. pénétrant dans les tissus pour y assujettir de façon permanente le dispositif médical.

Selon *un premier aspect,* l'invention concerne plus particulièrement ceux de ces dispositifs qui se présentent sous forme d'une capsule autonome implantée dans une cavité cardiaque (ventricule, oreillette ou même cavité cardiaque gauche artérielle), ci-après désignée "capsule autonome" ou "capsule *leadless"* (le caractère autonome de la capsule n'étant toutefois pas intrinsèquement une caractéristique nécessaire de l'invention). Ces capsules autonomes sont dépourvues de toute liaison physique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless",* pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

On verra par ailleurs que, selon *un second aspect,* l'invention peut être généralisée à la "délivrance", c'est-à-dire à la mise en place au site d'implantation choisi d'autres types de dispositifs médicaux, par exemple des sondes de stimulation se présentant sous forme d'un corps tubulaire portant à son extrémité distale des moyens d'ancrage à une paroi cardiaque ainsi qu'une partie active pourvue d'électrodes de détection/stimulation, et à son extrémité proximale des moyens de liaison mécaniques et électriques à un boitier de générateur, implanté à distance du site d'application des impulsions. L'invention peut s'appliquer à d'autres types encore de dispositifs implantables, par exemple à des capsules destinées à diffuser *in situ* d'un agent pharmacologique actif.

Dans le cas où les capsules *leadless* sont des capsules endocavitaires (c'est-à-dire des capsules à fixer à la paroi intérieure d'une cavité ventriculaire ou auriculaire, par opposition aux capsules épicardiques, fixées à la paroi extérieure du coeur), les contraintes d'implantation sont accrues du fait de la voie d'approche, qui implique de passer par le réseau veineux périphérique puis de diriger sous amplificateur de brillance la capsule vers le site d'implantation choisi, ceci de façon à la fois précise et parfaitement sécurisée. Ce n'est qu'une fois le site atteint et la capsule fermement ancrée dans la par paroi cardiaque que l'opérateur pourra procéder au "largage" de cette capsule, c'est-à-dire à sa désolidarisation d'avec l'accessoire d'implantation.

Le US 2012/0095539 A1 décrit un accessoire d'implantation d'une capsule *leadless* endocavitaire d'électrostimulation. Cet accessoire comprend un cathéter orientable supportant la capsule à son extrémité et logeant dans sa lumière interne un fil qui est relié côté distal à la capsule et qui est manoeuvrable en translation et en rotation depuis l'extrémité proximale au moyen d'une poignée à disposition du praticien. Dans un premier mode de réalisation, la capsule est montée à l'extrémité du cathéter par un système d'emboitement mâle/femelle et l'extrémité du fil est vissée à l'arrière de la capsule. Le fil de rétention permet de maintenir les deux éléments du système de couplage emboités l'un dans l'autre par une tension légère sur le fil, ce dernier étant bloqué en translation dans la poignée de manipulation. Dans un second mode de réalisation, le fil reste attaché à la capsule après que celle-ci a été séparée du cathéter, de manière à jouer un rôle de fil de sécurité au cas où il serait nécessaire de réintervenir sur la capsule postérieurement à son implantation.

Le EP 2 394 695 A1 (Sorin CRM SAS) décrit un autre ensemble de capsule intracardiaque autonome avec son accessoire d'implantation, dans lequel la capsule porte sur la paroi latérale de son corps tubulaire des doigts de couplage coopérant avec un guide hélicoïdal porté par l'extrémité distale de l'accessoire d'implantation. Le sens d'hélice du guide hélicoïdal est inverse de celui de la vis d'ancrage de la capsule, de manière à pouvoir transmettre le couple de vissage de la vis d'ancrage dans la paroi cardiaque puis, après que la face avant de la capsule est venue en appui contre cette paroi, la séparation progressive de la capsule d'avec l'accessoire d'implantation par la poursuite du vissage du cathéter, les doigts de couplage glissant entre les spires d'un ressort hélicoïdal de compression, l'effet limiteur de couple étant obtenu par la compression de ce ressort hélicoïdal.

L'acceptation par les praticiens de la technique des capsules *leadless* endocavitaires implique de pouvoir proposer un système de délivrance qui soit capable de sécuriser l'implantation de ces capsules en répondant à l'ensemble des contraintes suivantes :
- procédure proche de la pratique courante, qui fasse appel à des gestes bien connus et maitrisés des praticiens : ponction sous-clavière ou fémorale, insertion et manipulation d'un cathéter via des mandrins préformés pendant la phase d'approche du site d'implantation choisi, fixation de type vis ou barbe, manipulation de cathéter type électrophysiologie, etc. ;
- environnement standard du bloc opératoire ;
- limitation des risques de "carottage" des tissus du fait d'un vissage excessif qui pourrait endommager la paroi ou, pire, la perforer (notamment dans le cas de l'implantation dans une paroi mince telle que le septum interauriculaire ou la zone apicale du ventricule droit) ;
- possibilité de retrait et/ou de repositionnement per-opératoire ou postopératoire en cas de problème, même après largage de la capsule ;
- absence de risque de migration de la capsule en phase aigüe pendant l'intervention ;
- certitude d'une bonne fixation de la capsule avant retrait des accessoires d'implantation - cette contrainte étant la plus critique de toutes ;
- système nativement conçu pour un abord fémoral (voir ci-dessous) ;
- pour les vaisseaux et les cavités cardiaques, absence de risque d'endommagement par l'organe d'ancrage (vis, crochet, aiguille...) pendant toute l'opération d'implantation, y compris la navigation dans le réseau veineux et la phase d'approche jusqu'au site d'implantation choisi ;
- procédure rapide, avec un temps d'implantation cible d'environ 30 minutes "peau-à-peau", comparable à celui de l'implantation d'un générateur et d'une sonde ventriculaire conventionnelle ;
- fonctionnement sécurisé, y compris en cas i) de mauvaise manipulation, avec dans ce cas impossibilité de larguer la capsule si le vissage de l'organe d'ancrage est incomplet, et/ou possibilité de récupérer la capsule durant la procédure, et ii) d'abandon prématuré de la procédure ;
- faible cout de fabrication du système d'implantation complet, notamment par utilisation de technologies et de composants éprouvés dans des applications similaires.

Une difficulté supplémentaire se présente avec les capsules *leadless* actuelles du fait de leurs dimensions relativement importantes, avec un diamètre typique d'environ 4 à 7 mm pour une longueur de 15 à 40 mm.

En effet, pour accéder à une cavité cardiaque, et notamment atteindre le fond du ventricule droit, avec un objet d'une telle taille il n'existe pas de procédure routinière par voie haute, c'est-à-dire via la veine sous-clavière. Il est de ce fait nécessaire d'utiliser un abord différent, à partir d'une ponction fémorale puis en remontant la veine cave inférieure jusqu'au coeur.

Un tel abord fémoral est reconnu plus difficile, en particulier du fait de l'angulation importante entre la veine cave inférieure et l'axe du ventricule droit : en effet, dans le cas d'un abord par voie haute, à l'arrivée dans l'oreillette la partie distale du cathéter d'implantation se trouve naturellement orientée vers l'apex du ventricule droit, et il suffit de pousser le cathéter pour traverser la valve tricuspide et atteindre le fond du ventricule, où l'organe d'ancrage pourra être vissé après accostage à la paroi ; en revanche, dans le cas d'un abord fémoral, une fois atteinte l'oreillette il est nécessaire d'exécuter une manoeuvre de retournement de l'extrémité distale du cathéter pour orienter cette dernière vers le ventricule et lui permettre de traverser la valve tricuspide et poursuivre sa progression dans la bonne direction, vers l'apex du ventricule.

Il existe à cet effet des cathéters *steerables* bien connus dont l'extrémité distale est manoeuvrable depuis la poignée proximale de manière à pouvoir effectuer sous amplificateur de brillance une telle manoeuvre de réorientation dans l'oreillette. Mais une dernière difficulté subsiste lors de la phase finale d'approche, car la partie orientable du cathéter peut se révéler trop courte ou mal conformée pour permettre l'accostage avec la paroi de l'apex ventriculaire.

Il existe donc le besoin de pouvoir disposer d'un accessoire d'implantation permettant un ajustement fin et un abord précis du site d'implantation avec de grandes diversités d'anatomie du myocarde.

Pour résoudre notamment ce problème, l'invention propose, *selon un premier aspect,* d'utiliser un cathéter *steerable* et de prolonger celui-ci dans sa partie distale par un embout cylindrique de protection contenant la capsule à implanter. Cette capsule est maintenue en position rétractée dans l'embout via un sous-cathéter inséré dans la lumière interne du premier cathéter, la capsule et le sous-cathéter étant temporairement liés par un mécanisme débrayable simple fixé sur le sous-cathéter et permettant un vissage complet de la capsule dans les tissus avant largage. La configuration télescopique du sous-cathéter permet de projeter la capsule hors de l'embout de protection et au-delà de celui-ci sur plusieurs centimètres, autorisant en toutes circonstances une approche complète et précise de la capsule jusqu'au fond du ventricule.

*Selon un second aspect,* plus général, l'invention propose un mécanisme débrayable simple prévu entre un accessoire d'implantation et un dispositif médical (correspondant, respectivement, au sous-cathéter et à la capsule *leadless,* dans le cas particulier précédent). Ce mécanisme est constitué par un ressort hélicoïdal utilisé en compression radiale, c'est-à-dire pour son effet de striction, et non pour son effet de traction/compression axiale.

Un tel ressort peut jouer à la fois un rôle de moyen de liaison débrayable et de limiteur de couple à l'encontre d'une action de vissage excessive qui pourrait entraîner un "carottage" des tissus.

Plus précisément, selon le *premier aspect* précité, l'invention propose un ensemble de capsule intracardiaque avec son accessoire d'implantation *in situ,* du type divulgué par le EP 2 394 695 A1 précité, c'est-à-dire comprenant une capsule autonome avec un corps tubulaire cylindrique pourvu à son extrémité distale d'un organe d'ancrage apte à pénétrer dans un tissu d'une paroi d'une cavité du coeur, et un accessoire d'implantation de cette capsule. L'accessoire d'implantation comprend un cathéter avec une lumière interne, prolongé à son extrémité distale par un embout tubulaire de protection définissant un volume intérieur apte à loger la capsule, et des moyens découplables de support et de guidage de la capsule jusqu'à un site d'implantation.

De façon caractéristique de l'invention, le cathéter est un cathéter téléorientable, et l'accessoire d'implantation comprend en outre un sous-cathéter logé à l'intérieur de la lumière du cathéter téléorientable avec un degré de liberté en translation relative et un degré de liberté en rotation relative par rapport au cathéter téléorientable. Le sous-cathéter et la capsule sont déployables télescopiquement par rapport au cathéter téléorientable entre i) une position rétractée où la capsule et son organe d'ancrage sont complètement logés à l'intérieur de l'embout tubulaire de protection, et ii) une position déployée où la capsule est sortie de l'embout tubulaire de protection et est portée par l'extrémité distale du sous-cathéter. Enfin, l'extrémité distale du sous-cathéter et la région proximale de la capsule sont pourvues de premiers moyens de solidarisation en translation et en rotation mutuelles, ces moyens étant découplables sous l'effet d'une rotation appliquée au sous-cathéter depuis l'extrémité proximale de celui-ci.

Très avantageusement, l'accessoire d'implantation comprend en outre un fil de retenue logé dans une lumière du sous-cathéter et reliant la capsule à l'extrémité proximale du sous-cathéter. Le fil de retenue est mobile à l'intérieur de la lumière du sous-cathéter de manière à permettre le retrait de ce dernier en laissant en place la capsule et le fil de retenue. La région proximale de la capsule et l'extrémité distale du fil de retenue sont munies de seconds moyens découplables de solidarisation en translation et en rotation mutuelles.

Le fil de retenue peut notamment être un fil apte à transmettre un couple de rotation depuis son extrémité proximale jusqu'à son extrémité distale, et les seconds moyens de solidarisation comprennent des moyens à vis dissociables sous l'effet dudit couple de rotation appliqué au fil de retenue, ces moyens à vis comprenant un élément fileté formé à l'extrémité distale du fil de retenue et coopérant avec un élément taraudé formé à l'extrémité proximale de la capsule, ou *vice versa.*

Dans une forme de réalisation particulièrement préférée, les premiers moyens de solidarisation découplables comprennent un ressort hélicoïdal coopérant avec un noyau central, le ressort s'étendant autour du noyau avec un ajustement tel qu'il exerce sur celui-ci un effet de striction radiale, le ressort et le noyau pouvant être désolidarisés sous l'effet d'un couple de torsion appliqué au ressort à l'une de ses extrémités, ayant pour effet de réduire ladite striction radiale jusqu'à libération du noyau.

De préférence, l'extrémité proximale du ressort est solidarisée à l'extrémité distale du sous-cathéter, l'extrémité distale du ressort est libre, et le noyau est une tige d'arrimage axiale portée par l'extrémité proximale de la capsule et solidaire en rotation de cette dernière.

Lorsque la capsule porte une vis d'ancrage, le sens du ressort hélicoïdal est le même que celui de la vis d'ancrage.

La valeur du couple de torsion appliqué au ressort ayant pour effet de réduire ladite striction radiale jusqu'à libération du noyau est déterminée, en fonction de la géométrie du ressort et de l'élasticité du matériau qui le constitue, de manière à être toujours inférieure à une valeur limite prédéfinie, correspondant à un couple limite de maintien de la vis d'ancrage dans le tissu du site d'implantation, sans carottage de ce tissu.

D'autre part, pour permettre la mise en place de l'ensemble, il est possible d'utiliser un fil-guide, l'embout de protection comprenant alors dans l'épaisseur de sa paroi périphérique une lumière latérale s'étendant axialement et débouchant des deux côtés distal et proximal de l'embout, cette lumière étant apte à recevoir le fil-guide et à permettre le coulissement sur celui-ci de l'accessoire d'implantation avec la capsule logée dans l'embout de protection.

Enfin, l'embout de protection comporte de préférence au moins un trou de purge, et au moins un marqueur radio-opaque.

Selon le *second aspect* précité, l'invention propose un ensemble du type divulgué par le EP 2 394 695 A1 précité, c'est-à-dire comprenant un dispositif médical pourvu à son extrémité distale d'un organe d'ancrage apte à pénétrer dans un tissu d'une paroi d'un organe, et un accessoire d'implantation *in situ* du dispositif médical. Cet accessoire d'implantation comprend un élément tubulaire allongé déformable pourvu de moyens découplables de support et de guidage du dispositif médical jusqu'à un site d'implantation. Ces moyens découplables de support et de guidage comprennent un ressort hélicoïdal coopérant avec un noyau central et sont aptes à solidariser en translation et en rotation mutuelles l'élément tubulaire de l'accessoire d'implantation et le dispositif médical, et aptes à découpler le dispositif médical d'avec l'élément tubulaire de l'accessoire d'implantation sous l'effet d'une rotation appliquée à l'élément tubulaire depuis l'extrémité proximale de celui-ci.

De façon caractéristique, le ressort s'étend autour du noyau avec un ajustement tel qu'il exerce sur celui-ci un effet de striction radiale, le ressort et le noyau pouvant être désolidarisés sous l'effet combiné d'un couple de torsion et d'une traction appliqués au ressort à l'une de ses extrémités, ayant pour effet de réduire ladite striction radiale jusqu'à libération du noyau.

De préférence, l'extrémité proximale du ressort est solidarisée à l'extrémité distale de l'élément tubulaire de l'accessoire d'implantation, l'extrémité distale du ressort est libre, et le noyau est une tige d'arrimage axiale portée par une extrémité proximale du dispositif médical et solidaire en rotation de ce dernier.

Lorsque le dispositif médical porte une vis d'ancrage, le sens du ressort hélicoïdal est le même que celui de la vis d'ancrage.

La valeur dudit couple de torsion appliqué au ressort ayant pour effet de réduire ladite striction radiale jusqu'à libération du noyau est déterminée, en fonction de la géométrie du ressort et de l'élasticité du matériau qui le constitue, de manière à être toujours inférieure à une valeur limite prédéfinie, correspondant à un couple limite de maintien de la vis d'ancrage dans le tissu du site d'implantation, sans carottage de ce tissu.

Enfin, l'extrémité distale du ressort est avantageusement une extrémité arrondie.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre l'accessoire d'implantation de l'invention et sa capsule, en situation, avec une représentation schématique de la voie d'accès fémorale et des cavités du coeur.
La Figure 2 montre l'extrémité distale du cathéter téléorientable muni de son embout de protection, en position rétractée de la capsule *leadless.*
La Figure 3 est une vue agrandie et en coupe de la Figure 2, montrant la configuration générale des différents éléments internes ainsi que la capsule *leadless* logée à l'intérieur de l'embout de protection.
La Figure 4 est une vue agrandie de la coupe de la Figure 3, dans la région de la liaison avec l'extrémité proximale de la capsule *leadless.*
La Figure 5 montre isolément le ressort hélicoïdal de compression radiale, monté à l'extrémité du sous-cathéter.
La Figure 6 est une vue en coupe correspondant à la Figure 5.
La Figure 7 montre l'extrémité distale du cathéter téléorientable muni de son embout de protection, monté sur un fil-guide spiralé utilisé pour faire progresser cet embout depuis la ponction fémorale jusqu'au site d'implantation choisi.
La Figure 8 montre l'extrémité distale du cathéter téléorientable muni de son embout de protection, avec la capsule *leadless* partiellement émergée de cet embout.
La Figure 9 est une vue agrandie et en coupe de la Figure 8, montrant la configuration générale des différents éléments internes.
La Figure 10 est une vue agrandie et en coupe homologue de la Figure 9, une fois la capsule implantée au site choisi, après retrait du sous-cathéter et avec le fil de retenue toujours en place.
La Figure 11 montre la configuration finale de la capsule et de l'accessoire d'implantation, dans la situation correspondant à celle de la Figure 10.

On va maintenant décrire un exemple de réalisation du dispositif de l'invention.

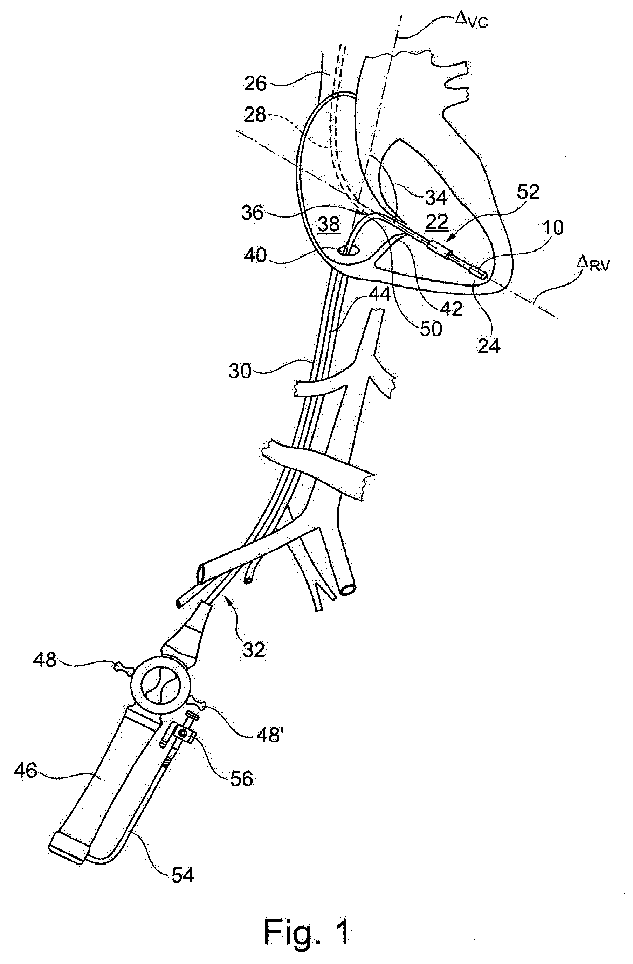

La Figure 1 illustre en situation, avec une représentation schématique de la voie d'accès fémorale et des cavités du coeur, l'accessoire d'implantation de l'invention portant une capsule autonome de type *leadless* référencée 10.

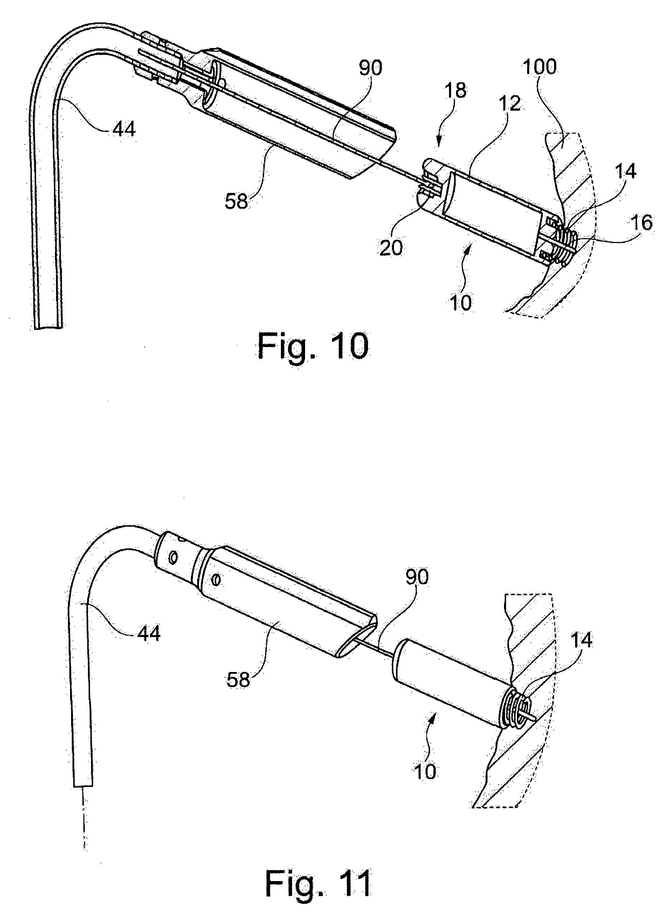

Une telle capsule *leadless* (représentée plus en détail notamment sur la Figure 10) comprend de façon en elle-même connue un corps tubulaire 12 pourvu à l'une de ses extrémités d'une vis d'ancrage 14 hélicoïdale saillante prolongeant axialement le corps tubulaire et solidaire de celui-ci en rotation. La vis d'ancrage comprend dans sa partie distale une longueur de l'ordre de 1,5 à 2 mm de spires non jointives, destinées à pénétrer dans le tissu cardiaque de façon à y assujettir la capsule. La vis 14 peut être une vis électriquement active, c'est-à-dire jouant, au moins à son extrémité distale, le rôle d'électrode de détection/stimulation, ou bien une vis passive ne servant qu'à l'ancrage du corps tubulaire 12 dans la paroi de la cavité cardiaque. Dans ce dernier cas, la capsule est munie d'une aiguille axiale conductrice 16 jouant le rôle d'électrode de détection/stimulation en contact avec les tissus du myocarde. En variante, il est également possible de prévoir une électrode de surface.

Le corps tubulaire 12 inclut divers moyens et circuits d'alimentation, de traitement du signal et de communication sans fil pour permettre l'échange de signaux avec un dispositif maitre distant, implanté ou non. Ces aspects sont en eux-mêmes connus et, comme ils ne font pas partie de l'invention, ils ne seront pas décrits.

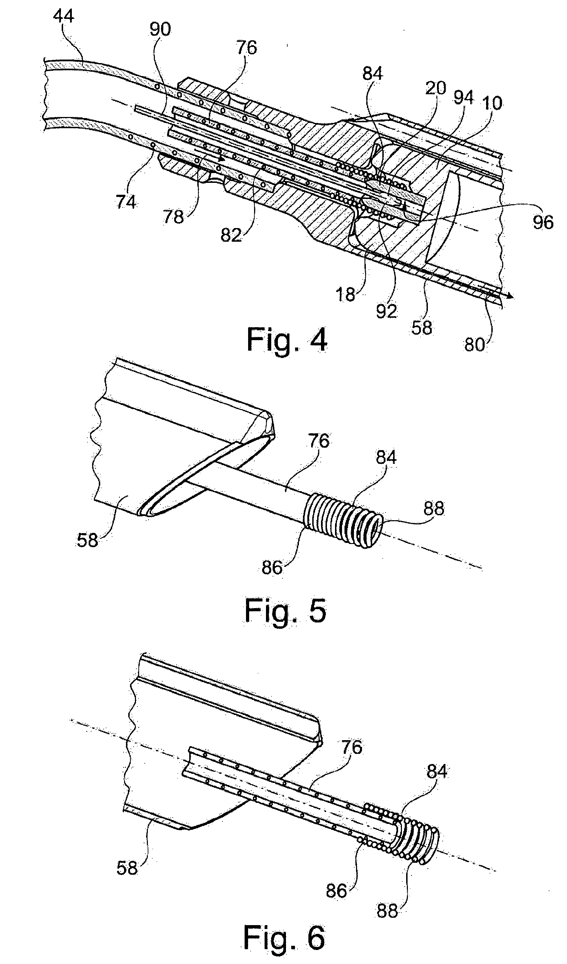

À son extrémité proximale 18, le corps tubulaire 12 de la capsule 10 comprend une tige d'arrimage axial 20 à extrémité arrondie dont on décrira plus loin le rôle lors de la procédure d'implantation. Cette tige d'arrimage 20 est lisse sur sa face extérieure et comporte un orifice axial taraudé interne, structure qui sera exposée plus en détail avec la description de la Figure 4 ci-après.

La capsule *leadless* 10 est destinée à être implantée dans le ventricule droit 22, notamment au fond de ce ventricule, dans la région de l'apex 24. Pour une sonde de stimulation conventionnelle (reliée à un générateur distant), l'implantation se ferait typiquement par voie haute via la veine sous-clavière 26, comme illustré en tiretés en 28, de sorte que l'extrémité de la sonde se trouverait approximativement orientée dans l'axe Δ_{RV} du ventricule droit et pourrait ainsi aisément traverser la valve tricuspide et parvenir à l'apex 24 du ventricule.

En revanche, comme on l'a indiqué en introduction, cette voie d'approche n'est pas envisageable pour l'implantation de capsules *leadless,* dont les dimensions, et notamment le diamètre externe, sont très supérieures à ceux d'une tête de sonde conventionnelle.

Il est de ce fait nécessaire de procéder par voie basse, via la veine cave 30, depuis une ponction fémorale 32. Mais dans ce cas, l'axe d'approche, c'est-à-dire l'axe Δ_{VC} de la veine cave, présente une très forte angulation (angle 34) avec l'axe Δ_{RV} du ventricule droit, nécessitant de ce fait une manoeuvre pour former une courbure 36 au niveau de l'oreillette droite 38 afin de pouvoir faire passer l'accessoire d'implantation émergeant du sinus 40 de la veine cave jusqu'à la valve tricuspide 42 pour atteindre ensuite la cavité du ventricule droit 22.

Des difficultés comparables se présentent pour une implantation dans le ventricule gauche, la voie d'accès impliquant alors une ponction fémorale artérielle et le passage de la crosse aortique.

Une telle manoeuvre peut être exécutée au moyen d'un cathéter téléorientable ("*steerable*"), qui est un accessoire en lui-même connu et tout à fait conventionnel comportant un tube de cathéter 44 manipulé depuis l'extrémité proximale par une poignée de manoeuvre 46 à la disposition du praticien. À l'aide des manettes 48, 48' celui-ci peut créer et régler une courbure 50 permettant d'orienter l'extrémité distale 52 du cathéter 44 de façon précise, typiquement avec une orientation pouvant aller jusqu'à 180° dans les deux directions avec un rayon de courbure variable, de l'ordre de 5 à 60 cm. La poignée 46 est également pourvue d'une voie latérale 54 de purge ainsi que d'une vanne de purge 56, caractéristiques en elles-mêmes tout à fait conventionnelles.

Avec un cathéter téléorientable conventionnel, s'il est possible d'ajuster avec précision la courbure 50, il n'est pas possible de modifier la zone du cathéter où, sur sa longueur, cette courbure se forme. Or, dans le cas particulier illustré d'un abord fémoral avec comme cible le fond du ventricule droit, cette limitation peut être gênante avec certaines morphologies particulières présentant une cavité très allongée : en effet, la partie distale 52 du cathéter téléorientable située au-delà de la région 50 de la courbure peut s'avérer trop courte pour atteindre la région de l'apex 24.

L'invention permet de pallier cette difficulté, comme on va l'expliquer ci-après, de sorte que la mise en oeuvre de l'invention est possible au moyen d'un cathéter téléorientable conventionnel du commerce, préexistant, permettant de réduire d'autant le cout de réalisation de l'accessoire d'implantation de l'invention.

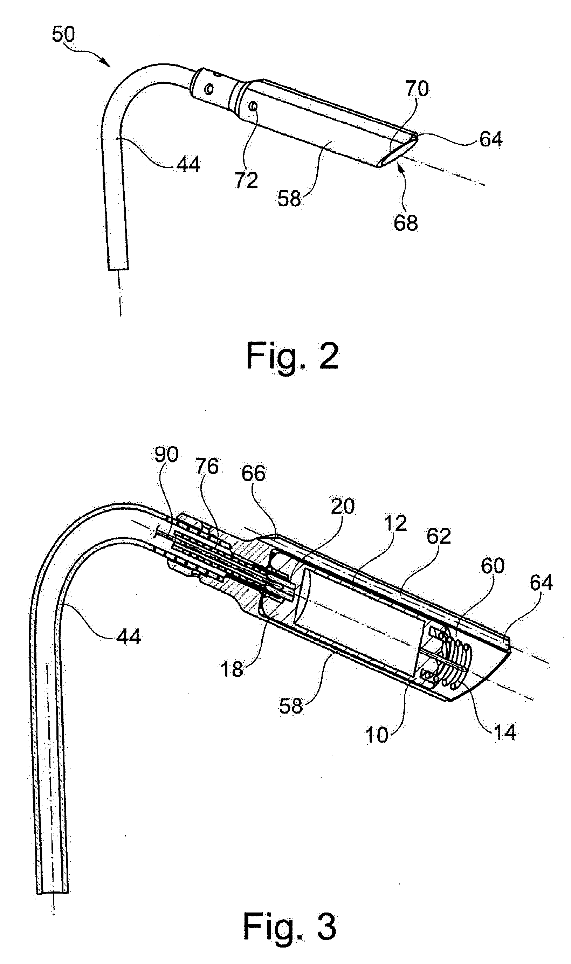

Les Figures 2 à 4 montrent, sous forme plus ou moins agrandie, l'extrémité distale du cathéter téléorientable 44, muni des différents éléments caractéristiques de l'invention.

Le cathéter téléorientable 44 est muni à son extrémité distale d'un embout tubulaire de protection 58 comportant un logement central 60 (Figure 3) permettant d'y loger la capsule 10 dans une configuration dite "position rétractée", correspondant aux Figures 2 à 4. La principale fonction de l'embout 58 est de protéger la capsule, et notamment la vis d'ancrage 14, pendant la montée intraveineuse au passage des virages, angulations, valves, etc. Réciproquement, l'embout protège les tissus des risques de *stripping* potentiellement occasionnés par le mouvement de translation de la vis.

Le diamètre extérieur du cathéter téléorientable 44 est typiquement compris entre 10 et 15 French (6,6 à 10 mm), pour un diamètre intérieur de lumière compris entre 8 et 12 French (2,66 à 4 mm). Quant à l'embout tubulaire 58, il doit pouvoir loger la capsule, donc présenter un diamètre intérieur de l'ordre de 21 French (7 mm)

Par ailleurs, un cathéter d'une telle taille doit nécessairement progresser dans le réseau veineux en étant guidé par un fil-guide spiralé préalablement introduit dans la vasculature.

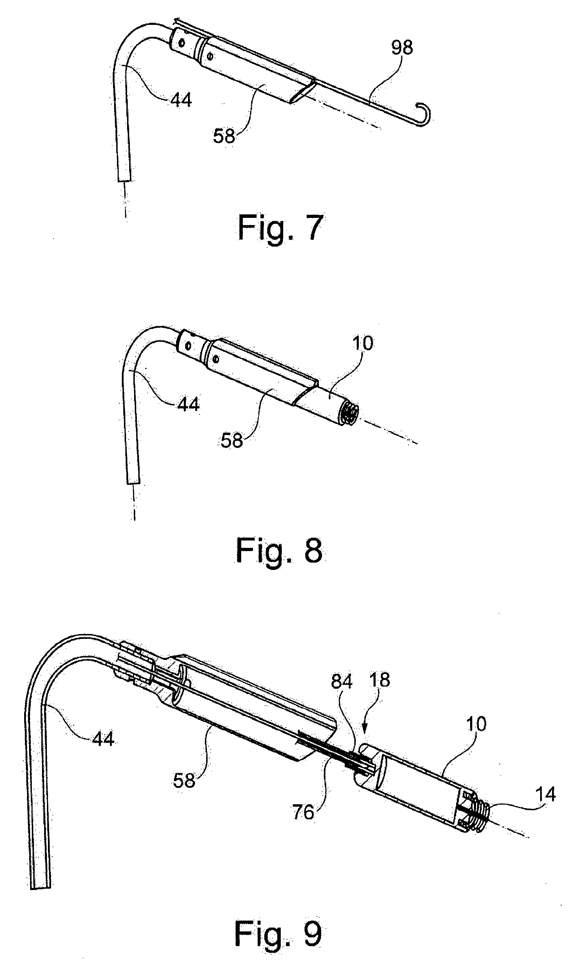

Comme, dans la conception illustrée, le canal central du cathéter 44 est obstrué par la capsule, pour permettre la mise en place d'un fil-guide l'embout tubulaire 58 est muni d'une lumière latérale excentrée 62 s'étendant axialement sur la longueur de l'embout et débouchant des deux côtés distal 64 et proximal 66, de préférence en s'étendant sur toute la longueur de l'embout 58. Le diamètre intérieur de cette lumière latérale 62 permet l'introduction d'un fil-guide spiralé conventionnel de diamètre 3 French (1 mm), et le coulissement de l'embout, et donc de l'ensemble du cathéter téléorientable 44, à travers la vasculature (cette configuration es notamment illustrée Figure 7, où la référence 98 désigne le fil-guide spiralé).

En variante, la lumière latérale excentrée 62 peut être prolongée tout le long du corps du cathéter téléorientable 44, afin de faciliter la poussée du guide spiralé et éviter tout phénomène de bouclage de ce dernier.

On notera que l'excentration de la lumière 62 combinée au profil biseauté de l'embout permet une progression aisée dans le réseau veineux par une technique *"sidewire".* Par ailleurs, la face avant 68, la plus distale, de l'embout 58 est profilée de manière à présenter une surface d'appui frontal minimale pour éviter tout risque de perforation en cas de manipulation accidentelle sans guide spiralé.

De plus, un marqueur radio-opaque 70 est prévu à l'avant de l'embout tubulaire 58 sur la face la plus distale de cet embout, afin d'identifier plus efficacement la sortie de la capsule si l'embout est réalisé dans un matériau radio-transparent.

Enfin, un ou plusieurs trous de purge 72 sont disposés côté proximal de l'embout, pour éviter tout effet de piston lors d'une injection de produit de contraste, qui sinon aurait pour conséquence de tendre à repousser la capsule 10 hors de l'embout de protection 58.

Le cathéter 44 est réalisé, de manière en elle-même connue, avec une structure armée, telle qu'un treillis métallique ou un bobinage noyé dans l'épaisseur de la paroi du cathéter, de manière à procurer une capacité de transmission du couple exercée sur la poignée de manoeuvre proximale jusqu'à l'extrémité distale (structure armée 74).

L'accessoire d'implantation de l'invention comprend en outre, de façon caractéristique, un sous-cathéter 76, introduit dans la lumière centrale du cathéter téléorientable 44, et mobile en rotation et en translation par rapport à ce dernier. La fonction de ce sous-cathéter 76 est d'assurer le déploiement de la capsule hors de l'embout de protection et d'avancer cette capsule jusqu'au site d'implantation par un mouvement de translation sur une longueur suffisante, typiquement de 2 à 6 cm en fonction de l'anatomie du patient (la flèche 78 indique la translation du sous-cathéter 76 à l'intérieur du cathéter téléorientable 44, et la flèche 80 indique la translation de la capsule 10 hors de l'embout de protection 58).

Le sous-cathéter 76 a également pour fonction d'assurer la transmission d'un couple de rotation depuis l'extrémité proximale (au niveau de la poignée de manoeuvre) jusqu'à son extrémité distale, et il est muni à cet effet d'une structure armée 82.

Il est possible d'utiliser pour le sous-cathéter 76 un cathéter-guide conventionnel de 4 à 6 French (1,33 à 2 mm), qui est un dispositif existant, simple et économique, répondant aux contraintes courantes de transmission du couple, de faible coefficient de frottement à l'intérieur et à l'extérieur, de flexibilité, etc., et qui dispose d'une connectique "Luer-Lok" proximale permettant le montage rapide d'un adaptateur multifonction tel qu'une valve hémostatique rotationnelle ou autre adaptateur compatible avec ce standard de connexion étanche. Subsidiairement, le sous-cathéter 76 permet d'injecter un produit de contraste jusqu'à l'arrière de la capsule 10 de manière à suivre avec précision l'opération sous amplificateur de brillance.

On va maintenant décrire en détail le moyen de couplage du sous-cathéter 76 à la capsule 10.

Ce moyen de couplage représente en soi un aspect original de l'invention, généralisable au couplage d'un dispositif d'implantation comprenant un élément tubulaire allongé creux (comme un cathéter) ou non, avec un dispositif médical autonome (comme une capsule *leadless*) ou non (comme une tête de sonde d'une sonde de stimulation), ce dispositif étant pourvu à son extrémité distale d'un organe d'ancrage apte à pénétrer dans un tissu corporel, cardiaque ou autre.

Le moyen de couplage selon l'invention met en oeuvre un ressort hélicoïdal 84 qui est utilisé non pas pour ses propriétés d'élasticité en traction/compression axiale (effet résultant de l'allongement ou du rapprochement des spires du ressort), mais pour ses propriétés de compression radiale, c'est-à-dire pour l'effet de striction ou étranglement qu'un tel ressort peut exercer autour d'un noyau central introduit à l'intérieur de la forme hélicoïdale.

La géométrie du ressort et l'élasticité du matériau qui le constitue sont choisies de manière à réaliser entre le ressort et le noyau central, en l'absence de sollicitation extérieure, un ajustement avec serrage (la compression radiale résultant de l'effet de striction).

Dans l'exemple illustré Figure 4 de la capsule *leadless* 10, le noyau est constitué par la tige d'arrimage à extrémité arrondie 20, située axialement sur la partie proximale 18 de la capsule 10 et orientée vers l'extérieur. Cette tige d'arrimage 20 peut être profilée pour optimiser la fonction de débrayage.

Ce ressort 84 est illustré plus précisément et de façon isolée sur les Figures 5 et 6.

Le ressort 84, quant à lui, est solidarisé à l'extrémité distale du sous-cathéter 76 par des spires 86. Cette solidarisation en translation et en rotation, par exemple par soudage ou collage, devra être conservée quel que soit le degré des contraintes normalement appliquées au sous-cathéter 76 et au ressort 84.

Les spires 88 situées côté distal du ressort 84 sont, en revanche, des spires libres, qui viennent entourer la tige d'arrimage 20 mais qui ne sont pas solidarisées mécaniquement à cette dernière autrement que par l'ajustement avec serrage obtenu dans la configuration statique de ces deux éléments. Par ailleurs, l'extrémité distale du ressort 84 est avantageusement une extrémité arrondie, afin d'éviter de blesser les tissus et de s'accrocher lors des différentes manipulations.

Les spires inactives 86 et/ou les spires actives 88 peuvent être indifféremment jointives ou non jointives.

Une fois que la capsule 10 est immobilisée au site d'implantation après pénétration complète de la vis d'ancrage 14 jusqu'à la face avant de la capsule, le praticien continue de faire tourner le sous-cathéter 76, générant ainsi un surcouple qui a pour effet de diminuer l'effort de striction exercé par les spires libres 88 sur la tige d'arrimage 20, jusqu'à provoquer le glissement en rotation de ces spires sur cette même tige. En combinant à ce mouvement de rotation un léger effort de traction, le ressort de compression 84 se dégage de la tige d'arrimage 20, libérant ainsi la capsule 10 d'avec le ressort 84, et donc d'avec le sous-cathéter 76.

Le ressort de compression radiale 84 joue ainsi un rôle de limiteur de couple. En effet, avec une vis d'ancrage d'une capsule *leadless* standard, si le praticien poursuivait la rotation du sous-cathéter 76 et donc de la capsule 10, le couple augmenterait et dépasserait une limite C_{carottage}, la vis d'ancrage risquant alors de déchirer localement les tissus sous l'effet de la rotation de la vis sans avance de celle-ci, jusqu'à provoquer une lacération des tissus et, à l'extrême, une perforation de la paroi avec risque de tamponnade. Tel n'est pas le cas avec le moyen proposé par l'invention : le praticien peut en effet poursuivre sans risque la rotation du sous-cathéter 76, toujours dans le même sens (généralement le sens horaire), car le couple supplémentaire apparaissant du fait de la réaction de la vis d'ancrage ancrée dans les tissus est absorbé par la liaison entre le ressort 84 et la tige d'arrimage 20 (phénomène de montée brusque du couple retour lorsque la face avant de la capsule touche le tissu cardiaque). Plus précisément, la géométrie et l'élasticité du ressort 84 sont choisies de manière à définir un couple limite prédéfini C_{largage} inférieur à la limite C_{carottage} du carottage correspondant à un couple limite de maintien de la vis d'ancrage dans le tissu du site d'implantation, sans carottage de ce tissu, tout en garantissant un vissage complet (tissus en contact avec la face avant de la capsule). De la sorte, lorsque le couple C_{débrayage} est atteint, la poursuite de la rotation du sous-cathéter 76 dans le sens horaire provoque, en combinaison avec un léger effort de traction, le dégagement progressif du ressort 84 d'avec la tige d'arrimage 20 (s'il y a surcouple, les spires du ressort radial glissent en rotation sur le noyau d'arrimage donc ne transmettent plus d'élévation du couple).

Le couple de débrayage C_{débrayage} est par exemple ajusté à une valeur typique de l'ordre de 0,2 à 0,8 N.cm.

Par ailleurs, dans une configuration statique, l'effort de striction de la partie libre 88 du ressort 84 sur la tige d'arrimage 20 est choisi de manière à empêcher tout désassemblage accidentel par un effort de traction (effort orienté axialement) inférieur à un seuil suffisant, typiquement un seuil qui assure un maintien même pour une traction exercée sur le sous-cathéter 76 avec un effort allant jusqu'à 20 N.

On notera par ailleurs que si l'on souhaite dévisser la capsule, par exemple parce qu'après une première implantation on constate que les performances électriques du site ne sont pas satisfaisantes, le système de couplage par le ressort 84 ne produira aucun effet de débrayage au dévissage, car le ressort sera alors entrainé en rotation inverse (généralement dans le sens antihoraire) ce qui aura pour effet d'augmenter encore plus le serrage des spires 88 sur la tige d'arrimage 20.

Un autre avantage du ressort 84 est qu'après largage de la capsule le dispositif d'implantation se présentera à son extrémité sous la forme illustrée Figures 5 et 6, c'est-à-dire que les spires libres 88 du ressort 84 formeront une vis. En cas de réintervention per-opératoire, c'est-à-dire si l'on veut à nouveau arrimer le sous-cathéter 76 à la capsule, la vis formée par le ressort 84 présentera l'avantage de ne nécessiter aucun ajustement angulaire pour venir s'arrimer à la tige 20 de la capsule à récupérer (à la différence des systèmes utilisant un connecteur mâle/femelle qui nécessite un positionnement pour permettre l'emboitement des deux éléments).

Enfin, on notera que le limiteur de couple constitué par le ressort 84 est idéalement situé dans la chaine de transmission des efforts : plus précisément, toute perte de fidélité dans la transmission du couple entre l'extrémité proximale du sous-cathéter 76 (c'est-à-dire depuis la poignée manipulée par le praticien) et son extrémité distale (là où se trouve le ressort de couplage 84) est sans effet sur le couple maximal ou minimal subi à l'interface entre la vis d'ancrage et les tissus, ce qui est un gage d'une fixation complète - ce qui ne serait pas le cas pour un système débrayable situé plus en amont, typiquement dans la poignée de manipulation 46. On notera par ailleurs que l'ensemble de ces fonctionnalités est obtenu via un composant très économique, de conception très simple et très compact.

Le largage de la capsule peut ainsi s'effectuer par un mouvement combiné de vissage puis de traction, en deux étapes :
- vissage de la capsule dans la paroi cardiaque, par une rotation dans le sens horaire du sous-cathéter 76 (par exemple sur 10 tours), sous une légère poussée, puis
- largage de la capsule, par une poursuite de la rotation horaire du sous-cathéter 76 (par exemple sur 5 tours), sous une légère traction afin de permettre le retrait du sous-cathéter après desserrage du ressort 84.

Pour obtenir ce résultat, le sens des spires du ressort est bien évidemment choisi dans le même sens que celui de la vis d'ancrage, de préférence avec un pas à droite pour que le vissage de la capsule puis son largage correspondent à une rotation du sous-cathéter 76 dans le sens horaire, le plus conventionnel.

Avantageusement, le nécessaire d'implantation comprend également un fil de sécurité ou fil de retenue 90 de type "fil d'Ariane" relié à la capsule 10 du côté distal, s'étendant sur toute la longueur du sous-cathéter 76 et dépassant de ce dernier du côté proximal, c'est-à-dire du côté de la poignée de manoeuvre 46.

Comme illustré sur les Figures 10 et 11, une fois la capsule 10 implantée et larguée, son fonctionnement est testé, notamment le bon établissement de la communication sans fil entre la capsule et le dispositif maitre distant ainsi que les performances électriques de stimulation.

Une fois le cathéter orientable 44 et le sous-cathéter 76 complètement retirés, le fil de retenue permet de récupérer la capsule en per-opératoire, avec réintroduction de l'accessoire d'implantation en faisant glisser celui-ci le long du fil de retenue jusqu'à ce que l'embout de protection 58 vienne coiffer la capsule.

Cette dernière pourra alors être réaccouplée au sous-cathéter par une rotation dans le sens horaire (la fonctionnalité de limiteur de couple étant toujours efficace), puis dévissée de la paroi 100 par une rotation dans le sens antihoraire et replacée sur un autre site par le même principe que ce qui a été décrit plus haut, par une rotation dans le sens horaire du sous-cathéter.

Le fil de retenue est par exemple un fil de 1 French (0,43 mm) de diamètre comportant à son extrémité distale 92 un filetage 94 apte à coopérer avec un taraudage homologue 96 réalisé dans un alésage axial taraudé de l'axe d'arrimage 20 (Figure 4). Ce fil de retenue doit être suffisamment souple dans sa partie distale (6 à 8 cm) tout en étant capable de transmettre jusqu'à l'extrémité distale 92 un couple de dévissage résultant d'une rotation imprimée depuis l'extrémité proximale, au niveau de la poignée de manoeuvre. On notera que, du fait du diamètre très faible du système vissant 94, 96, le couple à exercer pour réaliser le dévissage est très réduit (de l'ordre de 0,02 N.cm), et ne risque en aucune façon d'entrainer en rotation la capsule 10, qui est fermement assujettie à la paroi cardiaque par la vis d'ancrage.

Le fil de retenue peut être coloré de couleurs différentes pour chacune des capsules implantées, de manière à identifier plus facilement la capsule concernée dans l'éventualité d'une réintervention.

La technique de l'invention procure donc une triple sécurité grâce au système de débrayage qui permet lors du largage de la capsule :
- de garantir un vissage complet de la capsule dans les tissus ;
- d'éviter tout carottage de la paroi cardiaque ; et
- de garantir au praticien de pouvoir en cas de difficulté récupérer après largage la capsule grâce au fil de retenue.

La procédure de mise en place de la capsule *leadless* au moyen de l'accessoire d'implantation que l'on vient de décrire comprend les étapes suivantes, qui sont chacune relativement conventionnelles et peuvent être aisément réalisées par un praticien sans nécessiter d'habilité particulière ni de manoeuvres supplémentaires :
- ponction fémorale, droite ou gauche, de manière à accéder à la veine cave inférieure 30 ;
- mise en place optionnelle d'un introducteur 23 French (7,66 mm) hémostatique en percutané ;
- insertion du cathéter téléorientable 44 sur un fil-guide spiralé (illustré en 98 sur la Figure 7), typiquement un fil-guide de 3 French (1 mm) sur lequel l'embout tubulaire 58, et donc le cathéter téléorientable 44 pourront coulisser et progresser jusqu'à l'oreillette droite 38 ;
- manoeuvre de retournement de l'extrémité du cathéter téléorientable 44 (comme illustré en 36 sur la Figure 1), puis introduction de l'embout 58 dans le ventricule droit ;
- sortie de la capsule 10 jusqu'à l'apex du ventricule par translation du sous-cathéter 76 dans le cathéter téléorientable 44 (configuration illustrée Figure 9) ;
- visualisation des parois cardiaques par injection de produit de contraste via le sous-cathéter ;
- positionnement fin de la capsule au site cible choisi, avec possibilité de translation une fois dans la cavité cardiaque par déploiement plus ou moins important du sous-cathéter 76 hors du cathéter téléorientable 44, permettant un ajustement fin convenant à une grande diversité d'anatomies ;
- vissage de la capsule dans la paroi cardiaque jusqu'à débrayage du ressort de compression radiale 84 ;
- séparation du sous-cathéter 76 d'avec la capsule 10, et retrait du sous-cathéter 76 hors du cathéter téléorientable 44 (configuration illustrée Figure 10) ;
- test électrique de la capsule ;
- retrait complet du cathéter téléorientable 44 et du sous-cathéter 76 ;
- largage définitif de la capsule, avec retrait du fil de retenue 90 par un dévissage à faible couple ; et
- fermeture du point de ponction.

## Revendications

1. Un ensemble de capsule intracardiaque avec son accessoire d'implantation *in situ,* comprenant :
- une capsule autonome (10), comprenant un corps tubulaire cylindrique (12) pourvu à son extrémité distale d'un organe d'ancrage (14) apte à pénétrer dans un tissu (100) d'une paroi d'une cavité du coeur ; et
- un accessoire d'implantation de la capsule, comprenant :
- un cathéter (44) avec une lumière interne, prolongé à son extrémité distale par un embout tubulaire de protection (58) définissant un volume intérieur (60) apte à loger la capsule, et
- des moyens découplables de support et de guidage de la capsule jusqu'à un site d'implantation,
**caractérisé en ce que** :
- le cathéter est un cathéter téléorientable (44), et
- l'accessoire d'implantation comprend en outre :
- un sous-cathéter (76) logé à l'intérieur de la lumière du cathéter téléorientable avec un degré de liberté en translation relative et un degré de liberté en rotation relative par rapport au cathéter téléorientable,
le sous-cathéter et la capsule étant déployables télescopiquement par rapport au cathéter téléorientable entre i) une position rétractée où la capsule et son organe d'ancrage sont complètement logés à l'intérieur de l'embout tubulaire de protection, et ii) une position déployée où la capsule est sortie de l'embout tubulaire de protection et est portée par l'extrémité distale du sous-cathéter, et
- l'extrémité distale du sous-cathéter et la région proximale (18) de la capsule sont pourvues de premiers moyens de solidarisation (20, 84) en translation et en rotation mutuelles, ces premiers moyens de solidarisation étant découplables sous l'effet d'une rotation appliquée au sous-cathéter depuis l'extrémité proximale de celui-ci.

2. L'ensemble de la revendication 1, dans lequel l'accessoire d'implantation comprend en outre :
- un fil de retenue (90) logé dans une lumière du sous-cathéter et reliant la capsule à l'extrémité proximale du sous-cathéter, le fil de retenue étant mobile à l'intérieur de la lumière du sous-cathéter de manière à permettre le retrait de ce dernier en laissant en place la capsule et le fil de retenue, la région proximale de la capsule et l'extrémité distale (92) du fil de retenue étant munis de seconds moyens de solidarisation (94, 96) en translation et en rotation mutuelles, ces seconds moyens de solidarisation étant découplables.

3. L'ensemble de la revendication 2, dans lequel le fil de retenue est un fil apte à transmettre un couple de rotation depuis son extrémité proximale jusqu'à son extrémité distale, et les seconds moyens de solidarisation comprennent des moyens à vis dissociables sous l'effet dudit couple de rotation appliqué au fil de retenue, ces moyens à vis comprenant un élément fileté (94) formé à l'extrémité distale (92) du fil de retenue et coopérant avec un élément taraudé (96) formé à l'extrémité proximale (18) de la capsule, ou *vice versa.*

4. L'ensemble de la revendication 1, dans lequel les premiers moyens de solidarisation découplables comprennent un ressort hélicoïdal (84) coopérant avec un noyau central (20), le ressort s'étendant autour du noyau avec un ajustement tel qu'il exerce sur celui-ci un effet de striction radiale, le ressort et le noyau pouvant être désolidarisés sous l'effet combiné d'un couple de torsion et d'une traction appliqués au ressort à l'une de ses extrémités, ayant pour effet de réduire ladite striction radiale jusqu'à libération du noyau.

5. L'ensemble de la revendication 4, dans lequel l'extrémité proximale (86) du ressort est solidarisée à l'extrémité distale du sous-cathéter, l'extrémité distale (88) du ressort est libre, et le noyau est une tige d'arrimage axiale (20) portée par l'extrémité proximale (18) de la capsule et solidaire en rotation de cette dernière.

6. L'ensemble de la revendication 4, dans lequel l'organe d'ancrage de la capsule comprend une vis d'ancrage (14), et le sens du ressort hélicoïdal (84) est le même que celui de la vis d'ancrage.

7. L'ensemble de la revendication 6, dans lequel la valeur dudit couple de torsion appliqué au ressort ayant pour effet de réduire ladite striction radiale jusqu'à libération du noyau est déterminée, en fonction de la géométrie du ressort et de l'élasticité du matériau qui le constitue, de manière à être toujours inférieure à une valeur limite prédéfinie, correspondant à un couple limite de maintien de la vis d'ancrage dans le tissu du site d'implantation, sans carottage de ce tissu.

8. L'ensemble de la revendication 1, dans lequel :
- le nécessaire comprend en outre un fil-guide (98) ;
- l'embout de protection comprend dans l'épaisseur de sa paroi périphérique une lumière latérale (62) s'étendant axialement et débouchant des deux côtés distal (64) et proximal (66) de l'embout, cette lumière étant apte à recevoir le fil-guide et à permettre le coulissement sur celui-ci de l'accessoire d'implantation avec la capsule logée dans l'embout de protection ; et
- l'embout de protection comporte dans sa région la plus distale une face avant biseautée (68).

9. L'ensemble de la revendication 1, dans lequel l'embout de protection comprend au moins un trou de purge (72).

10. L'ensemble de la revendication 1, dans lequel l'embout de protection comprend au moins un marqueur radio-opaque (70).

## Patentansprüche

1. Einheit aus einer intrakardialen Kapsel mit entsprechendem Zubehörteil zur Implantation *in situ,* bestehend aus
- einer autonomen Kapsel (10), bestehend aus einem röhrenförmigen zylindrischen Körper (12), der an seinem distalen Ende mit einem Verankerungselement (14) versehen ist, welches geeignet ist, in ein Gewebe (100) einer Wand eines Herzhohlraums einzudringen; und
- einem Zubehörteil zur Implantation der Kapsel, bestehend aus:
• einem Katheter (44) mit einem internen Lumen, der an seinem distalen Ende durch ein röhrenförmiges Schutzendstück (58) verlängert wird, welches einen Innenraum (60) umschließt, der geeignet ist, die Kapsel aufzunehmen, und
• entkuppelbaren Mitteln zum Halten und Führen der Kapsel bis zu einem Implantationsort,
**dadurch gekennzeichnet, dass**
- der Katheter ein fernausrichtbarer Katheter (44) ist, und
- das Implantationszubehörteil außerdem
• einen Nebenkatheter (76) enthält, der im Inneren des Lumens des fernausrichtbaren Katheters mit einem Freiheitsgrad in Bezug auf die relative Translation und mit einem Freiheitsgrad in Bezug auf die relative Rotation zum fernausrichtbaren Katheter gelagert ist,
wobei der Nebenkatheter und die Kapsel zwischen i) einer eingezogenen Position, in der die Kapsel und deren Verankerungselement komplett im Inneren des röhrenförmigen Schutzendstücks gelagert sind, und ii) einer ausgefahrenen Position, in der die Kapsel aus dem röhrenförmigen Schutzendstück herausragt und vom distalen Ende des Nebenkatheters gehalten wird, in Bezug auf den fernausrichtbaren Katheter teleskopisch ausgefahren werden können, und
- das distale Ende des Nebenkatheters und der proximale Bereich (18) der Kapsel mit ersten zueinander verschiebbaren und verdrehbaren Befestigungsmitteln (20, 84) versehen sind, wobei diese ersten Befestigungsmittel durch eine Drehung des Nebenkatheters von dessen proximalem Ende aus entkuppelt werden können.

2. Einheit nach Anspruch 1, bei der das Implantationszubehörteil außerdem
• einen Rückholdraht (90) enthält, der in einem Lumen des Nebenkatheters gelagert ist und die Kapsel mit dem proximalen Ende des Nebenkatheters verbindet, wobei der Rückholdraht im Inneren des Lumens des Nebenkatheters beweglich ist, so dass letzterer herausgezogen werden kann und die Kapsel und der Rückholdraht vor Ort verbleiben, wobei der proximale Bereich der Kapsel und das distale Ende (92) des Rückholdrahtes mit zweiten, zueinander verschiebbaren und verdrehbaren Verbindungsmitteln (94, 96) versehen sind, wobei diese zweiten Verbindungsmittel entkuppelt werden können.

3. Einheit nach Anspruch 2, bei der der Rückholdraht ein Draht ist, der geeignet ist, von seinem proximalen Ende aus bis zu seinem distalen Ende ein Drehmoment zu übertragen, und die zweiten Verbindungsmittel Schraubelemente enthalten, die mittels des genannten, auf den Rückholdraht ausgeübten Drehmoments voneinander getrennt werden können, wobei diese Schraubelemente ein Außengewindeelement (94) umfassen, das am distalen Ende (92) des Rückholdrahtes aufgebracht ist und mit einem Innengewindeelement (96) zusammenwirkt, das am proximalen Ende (18) der Kapsel aufgebracht ist, oder *umgekehrt.*

4. Einheit nach Anspruch 1, bei der die ersten entkuppelbaren Verbindungsmittel eine Spiralfeder (84) enthalten, die mit einem zentralen Kern (20) zusammenwirkt, wobei die Feder den Kern mit einer solchen Passung umschließt, dass sie einen radialen Einschnüreffekt auf diesen ausübt, wobei die Feder und der Kern durch die kombinierte Wirkung eines Torsionsmoments und einer Traktion, die auf die Feder an einem ihrer Enden ausgeübt werden und wodurch die genannte radiale Einschnürung bis zur Freigabe des Kerns verringert wird, voneinander getrennt werden können.

5. Einheit nach Anspruch 4, bei der das proximale Ende (86) der Feder mit dem distalen Ende des Nebenkatheters verbunden ist, das distale Ende (88) der Feder frei ist und der Kern ein axialer Befestigungsstift (20) ist, der vom proximalen Ende (18) der Kapsel gehalten wird und mit letzterer rotationsschlüssig verbunden ist.

6. Einheit nach Anspruch 4, bei der das Verankerungselement der Kapsel eine Verankerungsschraube (14) enthält und die Spiralfeder (84) und die Verankerungsschraube die gleiche Windungsrichtung haben.

7. Einheit nach Anspruch 6, bei der der Wert des genannten Torsionsmoments, das auf die Feder ausgeübt wird und die Verringerung der genannten radialen Einschnürung bis zur Freigabe des Kerns bewirkt, in Abhängigkeit von der Geometrie der Feder und der Elastizität des Materials, aus dem diese besteht, so bestimmt wird, dass er stets unter einem vordefinierten Grenzwert liegt, der einem Grenzhaltemoment der Verankerungsschraube im Gewebe des Transplantationsortes entspricht, ohne dass dieses Gewebe vollständig durchbohrt wird.

8. Einheit nach Anspruch 1, bei der
- das Zubehör außerdem einen Führungsdraht (98) enthält;
- das Schutzendstück in der Dicke seiner Außenwand ein seitliches Lumen (62) enthält, das axial verläuft und jeweils in die distale (64) und proximale (66) Seite des Endstücks mündet, wobei dieses Lumen geeignet ist, den Führungsdraht aufzunehmen und auf diesem das Implantationszubehörteil mit der im Schutzendstück gelagerten Kapsel zu verschieben; und
- das Schutzendstück in seinem distalsten Bereich eine abgeschrägte Vorderseite (68) enthält.

9. Einheit nach Anspruch 1, bei der das Schutzendstück mindestens eine Ablassöffnung (72) enthält.

10. Einheit nach Anspruch 1, bei der das Schutzendstück mindestens einen radio-opaken Marker (70) enthält.

## Claims

1. An intracardiac capsule unit with its accessory for implantation *in situ,* comprising:
- an autonomous capsule (10), comprising a cylindrical tubular body (12) provided at its distal end with an anchoring member (14) adapted to penetrate a tissue (100) of a heart cavity wall; and
- an accessory for implantation of the capsule, comprising:
. a catheter (44) with an internal lumen, extended at its distal end by a tubular protection tip (58) defining an internal volume (60) adapted to receive the capsule, and
. decouplable means for supporting and guiding the capsule up to an implantation site,
**characterized in that**:
- the catheter is a tele-orientable catheter (44), and
- the implantation accessory further comprises:
. a sub-catheter (76) received inside the lumen of the tele-orientable catheter with a relative translational degree of freedom and a relative rotational degree of freedom with respect to the tele-orientable catheter,
the sub-catheter and the capsule being telescopically extendable with respect to the tele-orientable catheter between i) a retracted position where the capsule and its anchoring member are fully received inside the tubular protection tip, and ii) an extended position where the capsule is out of the tubular protection tip and is carried by the distal end of the sub-catheter, and
- the distal end of the sub-catheter and the proximal region (18) of the capsule are provided with first means (20, 84) for mutual translational and rotational fastening, these first fastening means being decouplable under the effect of a rotation applied to the sub-catheter from the proximal end thereof.

2. The unit of claim 1, wherein the implantation accessory further comprises:
a retaining wire (90) received in a lumen of the sub-catheter and connecting the capsule to the proximal end of the sub-catheter, the retaining wire being movable inside the lumen of the sub-catheter so as to allow the removal of the latter by leaving in place the capsule and the retaining wire, the proximal region of the capsule and the distal end (92) of the retaining wire being provided with second means (94, 96) for mutual translational and rotational fastening, these second fastening means being decouplable.

3. The unit of claim 2, wherein the retaining wire is a wire adapted to transmit a rotational torque from its proximal end to its distal end, and the second fastening means comprise screwing means able to be dissociated under the effect of said rotational torque applied to the retaining wire, these screwing means comprising a threaded element (94) formed at the distal end (92) of the retaining wire and cooperating with a bored element (96) formed at the proximal end (18) of the capsule, or vice versa.

4. The unit of claim 1, wherein the first decouplable fastening means comprise an helical spring (84) cooperating with a central core (20), the spring extending around the core with such an adjustment that it exerts on the latter a radial necking effect, wherein the spring and the core are able to be unfastened from each other under the combined effect of a torsional torque and a traction applied to the spring at one of its ends, having for effect to reduce said radial necking up to the release of the core.

5. The unit of claim 4, wherein the proximal end (86) of the spring is fastened to the distal end of the sub-catheter, the distal end (88) of the spring is free, and the core is an axial stowing rod (20) carried by the proximal end (18) of the capsule and rotationally integral with the latter.

6. The unit of claim 4, wherein the anchoring member of the capsule comprises an anchoring screw (14), and the direction of the helical spring (84) is the same as that of the anchoring screw.

7. The unit of claim 6, wherein the value of said torsional torque applied to the spring having for effect to reduce said radial necking up to the release of the core is determined, as a function of the geometry of the spring and of the elasticity of the material of which it is consisted, so as to be always lower than a predefined limit value, corresponding to a limit torque for the holding of the anchoring screw in the tissue of the implantation site, with no coring of this tissue.

8. The unit of claim 1, wherein:
- the kit further comprises a guide-wire (98);
- the protection tip comprises, in the thickness of its peripheral wall, a lateral lumen (62) extending axially and opening at the two distal (64) and proximal (66) sides of the tip, this lumen being adapted to receive the guide-wire and to allow the sliding on the latter of the implantation accessory with the capsule received in the protection tip; and
- the protection tip includes in its most distal region a bevelled front face (68).

9. The unit of claim 1, wherein the protection tip comprises at least one draining hole (72).

10. The unit of claim 1, wherein the protection tip comprises at least one radiopaque marker (70).
